Europäisches Patentamt

European Patent Office

Office européen des brevets

(11) Publication number: **0 206 068 B1**

# EUROPEAN PATENT SPECIFICATION

(45) Date of publication of patent specification: **28.10.92**

(51) Int. Cl.⁵: **C12P 1/04**, C07G 11/00, A61K 35/66, C12N 1/20, //(C12P1/04,C12R1:39, C12N1:20,C12R1:39)

(21) Application number: **86107813.7**

(22) Date of filing: **07.06.86**

(54) Antibiotic TAN-749, production and use thereof.

(30) Priority: **18.06.85 JP 133491/85**
**23.12.85 JP 291055/85**

(43) Date of publication of application:
**30.12.86 Bulletin 86/52**

(45) Publication of the grant of the patent:
**28.10.92 Bulletin 92/44**

(84) Designated Contracting States:
**AT BE CH DE FR GB IT LI LU NL SE**

(56) References cited:

**No relevant documents have been disclosed.**

(73) Proprietor: **Takeda Chemical Industries, Ltd.**
**1-1, Doshomachi 4-chome**
**Chuo-ku, OSAKA(JP)**

(72) Inventor: **Harada, Setsuo**
**3-31, Seiwadainishi 2-chome**
**Kawanishi Hyogo 666-01(JP)**
Inventor: **Ono, Hideo**
**12-7, Uzumoridai 4-chome**
**Higashinada-ku Kobe Hyogo 658(JP)**

(74) Representative: **von Kreisler, Alek,**
**Dipl.-Chem. et al**
**Deichmannhaus am Hauptbahnhof**
**W-5000 Köln 1(DE)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

**Description**

The present invention relates to a novel antibiotic TAN-749 (abbreviated TAN-749 in some cases hereinafter), which can be used favorably as a therapeutic drug for bacterial infection and production thereof. Futhermore, the invention relates to a biologically pure culture producing one or more of this antibiotics.

TAN-749A and C and TAN-749B and D are expressed by the formulas, $C_{15}H_{27}N_5O_3$ and $C_{16}H_{29}N_5O_3$, respectively. Antibiotics possessing a similar molecular formula include leucylnegamycin (molecular formula: $C_{15}H_{31}N_5O_5$), which is produced by the microbe belonging to the genus Streptomyces, [The Journal of Antibiotics, 24, 732 (1971)].

Due to the development of therapeutics using antibiotics, bacterial diseases for the most part have been overcome; however, there are still some important problems in the field of infectious disease medicine. For example, long-term or high-dose medication with conventional antibiotics causes changes in the flora of disease-causative bacteria (replacement of bacteria) or the advent of drug-resistant bacteria (acquisition of drug-resistance), resulting in an increase in diseases; therapeutics for people lacking auto-immunity are much needed. Such antibiotics possessing a novel structure and thus novel biological activity, or intermediate materials for their synthesis, are needed to solve these problems.

The present inventors isolated a great number of bacterial species from the soil in search for new antibiotics and then separated and investigated antibiotics produced by those species, finding that some microbes produce a new antibiotic. These microbes belong to the genus Pseudomonas, capable of producing an antibiotic possessing antibacterial activity against both gram-positive and gram-negative bacteria including drug-resistant ones can be accumulated in a medium by incubating said microbes in the medium. The inventors then separated this antibiotic and on the basis of its physical, chemical and biological properties, proved that it was a new antibiotic; it was named antibiotic TAN-749. TAN-749 is composed of 4 constituents; these were named TAN-749A, B, C and D, respectively.

Therefore, the present invention relates to Antibiotics TAN-749A, B, C and D which possess the following physical and chemical properties as dihydrochlorides, or their salts.

TAN-749A

(1) Appearance: Colorless solid
(2) Molecular formula: $C_{15}H_{27}N_5O_3 \cdot 2HC\ell$
(3) Ultraviolet (UV) absorption spectrum (in water):

$$\lambda_{max} \ 266 \pm 3nm \ (E_{1cm}^{1\%} = 658 \pm 100)$$

(4) Infrared (IR) absorption spectrum (KBr method):
Main wavenumbers are as follows:
3400, 3100, 1700, 1670, 1550, 1440, 1380, 1340, 1280, 1220, 1150, 1100, 1000, 960, 870, 680 ($cm^{-1}$)
(5) $^{13}C$ nuclear magnetic resonance (NMR) spectrum:
The following signals are measured in deuterium oxide at 100 MHz:
174.7(s), 172.3(s), 171.7(s), 139.3(d), 139.3(d), 129.6(d), 125.3(d), 69.2(d), 49.2(d), 47.8(t), 39.8(t), 39.1(t), 38.1(t), 35.2(t), 16.0(q) (ppm)
(6) Color reaction:
    Positive:     Ehrlich's reaction, Dimethylbenzaldehyde, Potassium permanganate
    Negative:    Ninhydrin, Greig-Leaback's, Sakaguchi's, Dragendorff's reactions

TAN-749B

(1) Appearance: Colorless solid
(2) Molecular formula: $C_{16}H_{29}N_5O_3 \cdot 2HC\ell$
(3) UV spectrum (in water):

$$\lambda_{max} \ 264 \pm 3nm \ (E_{1cm}^{1\%} = 660 \pm 100)$$

(4) IR spectrum (KBr method):

3300, 3100, 1700, 1660, 1610, 1550, 1450, 1420, 1380, 1350, 1280, 1220, 1150, 1070, 1000, 960, 930, 870, 690 $(cm^{-1})$

(5) $^{13}C$ NMR spectrum:

The following signals are measured in deuterium oxide at 100 MHz:

174.7(s), 171.6(s), 139.3(d), 139.2(d), 129.6(d), 125.5(d), 72.6(d), 52.3(d), 49.3(d), 39.9(t), 39.1(t), 37.5(t), 35.2(t), 18.8(q) 16.0(q) (ppm)

(6) Color reaction:

    Positive:     Ehrlich's reaction, Dimethylbenzaldehyde, Potassium permanganate

    Negative:    Ninhydrin, Greig-Leaback's, Sakaguchi's, Dragendorff's reactions

TAN-749C

(1) Appearance: Colorless solid

(2) Molecular formula: $C_{15}H_{27}N_5O_3 \cdot 2HC\ell$

(3) UV spectrum (in water):

$$\lambda_{max} \ 262 \pm 3nm \ (E^{1\%}_{1cm} = 680 \pm 100)$$

(4) IR spectrum (KBr method):

3250, 3070, 1660, 1630, 1540, 1430, 1340, 1260, 1200, 1150, 1085, 995, 860, 650 $(cm^{-1})$

(5) $^{13}C$ NMR spectrum:

The following signals are measured in deuterium oxide at 100 MHz:

174.7(s), 172.3(s), 171.6(s), 145.1(d), 143.0(d), 132.0(d), 123.1(d), 69.2(d), 49.2(d), 47.7(t), 39.7(t), 39.1(t), 38.0(t), 35.2(t), 20.6(q) (ppm)

(6) Color reaction:

    Positive:     Ehrlich's reaction, Dimethylbenzaldehyde, Potassium permanganate

    Negative:    Ninhydrin, Greig-Leaback's, Sakaguchi's, Dragendorff's reactions

TAN-749D

(1) Appearance: Colorless solid

(2) Molecular formula: $C_{16}H_{29}N_5O_3 \cdot 2HC\ell$

(3) UV spectrum (in water):

$$\lambda_{max} \ 262 \pm 3nm \ (E^{1\%}_{1cm} = 655 \pm 100)$$

(4) IR spectrum (KBr method):

3250, 3050, 1660, 1635, 1530, 1435, 1345, 1260, 1200, 1150, 995, 860, 650 $(cm^{-1})$

(5) $^{13}C$ NMR spectrum:

The following signals are measured in deuterium oxide at 100 MHz:

174.7(s), 171.7(s), 171.6(s), 145.3(d), 143.0(d), 132.0(d), 123.2(d), 72.5(d), 52.2(d), 49.3.(d), 39.9(t), 39.2-(t), 37.5(t), 35.3(t), 20.7(q), 18.8(q) (ppm)

(6) Color reaction:

    Positive:     Ehrlich's reaction, Dimethylbenzaldehyde, Potassium permanganate

    Negative:    Ninhydrin, Greig-Leaback's, Sakaguchi's, Dragendorff's reactions

In the present specification, antibiotics TAN-749A, B, C and D, or each of them, is generally called antibiotic TAN-749 or simply TAN-749 in some cases.

Based on these findings, the inventors made further studies to complete the present invention.

The present invention relates to: (1) antibiotics TAN-749A, B, C and D, or their salts and (2) a method for producing antibiotics TAN-749A, B, C and D, or their salts, characterized in that microbes belonging to the genus Pseudomonas capable of producing one or more of antibiotics TAN-749A, B, C and D are incubated in a medium to produce and accumulate one or more of antibiotics TAN-479A, B, C and D in the culture broth and then to collect it (them) and (3) a pharmaceutical composition for treating bacterial infection, which contains an effective amount of one or more of antibiotics TAN-749 A, B, C and D, and a carrier.

Bacteria which can be used in the present method as microbes producing antibiotic TAN-749 include all microbes belonging to the genus Pseudomonas capable of producing antibiotic TAN-749, e.g. Pseudomonas fluorescens. To speak more concretely, included is Pseudomonas fluorescens YK-437 (abbreviated "strain YK-437" in some cases hereinafter), which was isolated from a plant collected at Mt. Shirouma, Nagano Prefecture, Japan.

Strain YK-437 has the following bacteriological characteristics:

(a) Morphology

Morphological characteristics were observed after incubation on a nutrient agar slant medium at 24°C for 5 days.

| | |
|---|---|
| Cell shape and size | : Rod, 0.5 ~ 1.0 $\mu$m × 1.5 ~ 4 $\mu$m |
| Motility | : Yes (flagellatory) |
| Sporulation | : No |
| Gram-stain | : Negative |
| Acid fastness | : Non-acid-fast |

(b) Growth states on various media

Growth states were observed on various media at 24°C for 1 to 14 days.

① Nutrient agar plate culture:

Colonies are colorless, transparent and circular.

The colony surface is head-like to convex spherical. The colony margin is sinuous. No diffusible pigment is produced.

② Nutrient agar slant culture:

Colonies are cloth-like, highly lustrous, opaque and colorless.

③ Nutrient broth culture:

Grows in turbid suspension. Forms a thin pellicle.

No precipitation appears.

④ Nutrient gelatin stab culture:

Grows well mainly on upper portion. Liquefies with high liquefication activity.

⑤ Litmus milk:

Litmus-reduction activity is not observed. Peptonization activity is observed but coagulation is not.

(c) Physiological characteristics

① Nitrate reduction: -

② Denitrification: -

③ MR (methyl red) test: -

④ VP (Voges-Proskauer) test: -

⑤ Indole production: -

⑥ Hydrogen sulfide production (lead acetate paper): -

⑦ Starch hydrolysis: -

⑧ Citric acid utilization (Kosel's citrate medium, Christensen's citrate medium, Simmons' citrate medium): +

⑨ Inorganic nitrogen source utilization:

    I) Potassium nitrate    : +

    II) Ammonium sulfate    : +

⑩ Pigment production (King's A medium, King's B medium, Mannitol yeast extract agar medium): Lemon-color intracellular pigment production and lemon-color water-soluble pigment production are both observed in King's B medium.

| | |
|---|---|
| King's A medium: | Glycerol 10g, Peptone 20g, Magnesium chloride 1.4g, Ammonium sulfate 10g, Agar 15g, Distilled water 1,000m$\ell$, pH 7.2 |
| King's B medium: | Glycerol 10g, Peptone 20g, Potassium monohydrogen phosphate 1.5g, Magnesium sulfate 1.5g, Agar 15g, pH 7.2 |

⑪ Urease: +

⑫ Oxidase: +

⑬ Catalase: +

⑭ Growing conditions:

    I) pH    : Grows in a pH range of 4.1 to 8.5. Optimum pH ranges from 6.3 to 8.2.

    II) Temperature    : Grows in a range of 8 to 36°C. Optimum temperature ranges from 11 to 24°C.

⑮ Oxygen demand: Aerobic

⑯ O-F (oxidative-fermentative) test (Hugh•Leifson method]: Oxidative

⑰ Acid and gas production from sugars and their utilization:

|  | Acid (Peptone Water) | Gas (Peptone Water) | Utilization (Davis'Medium) |
|---|---|---|---|
| L-arabinose | + | - | + |
| D-xylose | + | - | + |
| D-glucose | + | - | + |
| D-mannose | + | - | + |
| D-fructose | - | - | + |
| D-galactose | + | - | + |
| Maltose | - | - | + |
| Sucrose | ± | - | + |
| Lactose | - | - | + |
| Trehalose | - | - | + |
| D-sorbitol | - | - | + |
| D-mannitol | - | - | + |
| Inositol | - | - | + |
| Glycerol | - | - | + |
| Starch | - | - | + |
| +: Positive, ±: Pseudopositive, -: Negative | | | |

⑱ G + C (guanine-cytosine) content of DNA: 66.4 ± 1.5 mole%(Tm method)
⑲ Sodium chloride tolerance: 0 ~ 5%
⑳ Decomposing activity for carboxymethyl cellulose or colloidal chitin: -
㉑ Decomposing activity for agar or arginate: -
㉒ Decomposing activity for Tween 80: +

Strain YK-437 was collated with bacterial species described in Bergey's Manual of Determinative Bacteriology, 8th edition, or the International Journal of Systematic Bacteriology, Vol. 30, pp. 225~420 (1980) and validation lists shown in the journal; this strain was thought of as belonging to the genus Pseudomonas, based on the following facts, i.e. it is an aerobic gram-negative rod possessing flagellatory motility, it is positive in both catalase and oxidase activities, and the G + c content of its DNA is 66.4 ± 1.0 mole%.

According to said Bergey's Manual of Determinative Bacteriorogy, the genus Pseudomonas is divided into four sections, i.e. Sections I, II, III and IV, by its characteristics concerning requirement of growth factors, intracellular accumulation of poly-ß-hydroxy butyrate, utilization of DL-arginine and growth at 40°C.

Table 1 shows the characteristics of the strain YK-437 as obtained by further experiments.

Table 1

Characteristics of strain YK-437

| Test sort | Result* |
|---|---|
| Poly-β-hydroxybutyrate accumulation | - |
| Arginine dihydrolase | + |
| Pigment production | |
| King's A medium | - |
| King's B medium | + |
| Denitrification | - |
| Lipase (Tween 80) activity | - |
| Gelatin hydrolysis | + |
| Poly-β-hydroxybutyrate hydrolysis | - |
| Utilization of carbon sources** | |
| Trehalose | + |
| Sucrose | + |
| L-Arabinose | + |
| Propionate*** | - |
| Butyrate**** | - |
| Sorbitol | + |
| Adonitol | + |
| Propylene glycol | - |
| Ethanol | - |

* +: Positive, -: Negative
** Stanier's medium (Journal of General Microbiology Vol. 43, pp. 159-271 (1966)) was used.
*** Sodium propionate
**** Sodium butyrate

It was considered appropriate that the strain YK-437 belongs to the Section I on the basis of the facts that the strain has no auxotrophy and doesn't accumulate poly-β-hydroxybutyrate as an intracellular carbon reserve.

Ten species are included in Section I. As the strain YK-437 produces fluorochrome and possesses arginine-dihydrolase, the strain YK-437 was thought of as belonging to any of Pseudomonas aeruginosa, Pseudomonas putida, Pseudomonas fluorescens, Pseudomonas chlororaphis and Pseudomonas aureofaciens.

The strain YK-437 is different from Pseudomonas aeruginosa at the point of denitrification and utilization of trehalose and geraniol, and from Pseudomonas putida at the point of hydrolysis of gelatin and utilization of trehalose. The strain YK-437 is different from Pseudomonas chlororaphis at the point of denitrification, lipase-activity and utilization of carbon sources, and from Pseudomonas aureofaciens at the point of utilization of sorbitol and adonitol.

The above-described characteristics of the strain YK-437 are in good agreement with those of Pseudomonas fluorescens. Therefore, the strain YK-437 was identified with Pseudomonas fluorescens and was designated Pseudomonas fluorescens YK-437.

Pseudomonas fluorescens YK-437 has been deposited under the accession number of IFO 14446 at the Institute for Fermentation, Osaka (IFO), since June 7, 1985.

This microbe, which was deposited on June 15, 1985 at Fermentation Research Institute, Agency of Industrial Science and Technology, Ministry of International Trade and Industry, Japan (FRI) under the accession number of FERM P-8312, the deposit being converted to a deposit under the Budapest Treaty, has been stored at FRI under the accession number of FERM BP-1005.

Bacteria belonging to the genus Pseudomonas used in the method In this invention, in general, are very susceptible to mutagens, e.g., it can be varied easily by artificial variation using ultraviolet, X-rays, chemicals such as nitrosoguanidine and ethyl methanesulfonate, etc.; however, strains which can be used in the present invention include all variants capable of producing TAN-749.

In the incubation of TAN-749-producing bacteria, substances which can be assimilated by the bacteria are used properly as carbon sources: glucose, maltose, lactose, blackstrap molasses, oil and fats (soybean oil, olive oil, etc.), and organic acids (citric acid, succinic acid, gluconic acid, etc.). As nitrogen sources, various organic or inorganic nitrogen compounds can be used: soybean flour, cotton seed powder, corn steep liquor, dried yeast, yeast extract, meat extract, peptone, urea, ammonium sulfate, ammonium nitrate, ammonium chloride, and ammonium phosphate. Inorganic salts such as sodium chloride, potassium chloride, calcium carbonate, magnesium sulfate, potassium primary phosphate and potassium secondary phosphate, which are essential to ordinary bacterial cultures, can be properly used singly or in combination.

Heavy metals such as ferrous sulfate and copper sulfate, and vitamins such as vitamin $B_1$ and biotin, are added if required. Antifoaming agents such as silicon oil and polyalkylene glycol ether, and surface active agents, can also be added to the medium. Other organic or inorganic substances which facilitate bacterial growth and thus promote TAN-749 production can also be added.

As for culture methods, ordinary production methods for antibiotics can be applied; solid and liquid cultures are both applicable. In the case of liquid cultures, any of standing cultures, shaking cultures, submerged culture, aeration cultures, etc. can be conducted; the aeration submerged culture is especially preferable. Culture temperature can be chosen in a range of approx. 10°C to 30°C; it is recommended that the incubation be conducted between approx. 17°C and 24°C. A medium pH can be chosen in a range of approx. 4 to 8; recommended pH ranges from approx. 6 to 7. The culture should be performed for approx. 8 to 168 hours, preferably approx. 24 to 144 hours.

In collecting TAN-749, the purposed product, from cultures, separation methods which are usually employed to collect metabolites produced by microbes from their cultures can properly be used. For example, TAN-749, which behaves as a water-soluble alkaline substance, and is thus contained mainly in culture filtrate, can be collected by performing the following procedures. That is, the culture liquid, after addition of a filter aid, is subjected to filtration or centrifugation to remove bacterial cells. The resulting culture filtrate is put in contact with a proper carrier to adsorb biologically active components in the filtrate, and the active components are then desorbed using an appropriate solvent to separate and recover the purposed products. Chromatographic carriers which can be used favorably include compounds with which adsorbability difference is applied, such as activated charcoal, powdered cellulose and adsorptive resins, those with which functional group difference is applied, such as cation exchange resins, cation exchange cellulose and cation exchange dextran gel, and those with which a molecular weight difference is applied, such as dextran gel. Eluents which can be used in proper combination to elute purposed compounds from these carriers include hydrated solutions of water-soluble organic solvents, such as hydrated acetone and hydrated alcohols, and aqueous solutions containing acids, alkalis, buffer solutions, organic salts or inorganic salts, though combination varies with carrier types and qualities.

In some cases, crude products containing antibiotics, obtained using these chromatographic methods, are subjected to HPLC for separation to obtain purified products.

Methods for recovering TAN-749 are described in more detail hereinafter. Antibacterial substances contained in the filtrate can be adsorbed using cation-exchange resins such as Amberlite IRC-50 and CG-50 (Rohm & Haas Co., USA) and then eluted using aqueous solutions or buffer solutions containing salts or acids. Antibiotics can be adsorbed also using cation-exchange dextran gels such as CM-Sephadex (Pharmacia Fine Chemicals, Sweden) and then eluted using aqueous or buffer solutions containing salts or acids. It is recommended that activated charcoal for chromatography (Takeda Chemical Industries Co., Ltd., Japan) or adsorptive resins such as Diaion HP-20 and SP-207 (Mitsubishi Chemical Industries Co., Ltd., Japan) and Amberlite XAD-II (Rohm & Haas Co., USA) be used to remove salts and coloring substances, etc. from resulting eluates. Eluted fractions are powdered via processes including concentration and lyophilization. When the resulting powder is low in purity, the use of HPLC is recommended for further purification. Carriers which can be used in such HPLC include TSK gel (Toyo Soda Manufacturing Co., Ltd.,

Japan) and YMC gel (Yamamura Chemical Laboratories, Japan). As for mobile phase, mixed solutions of methanol, acetonitrile, etc. and an aqueous or buffer solutions containing inorganic salts, can be used. TAN-749 is separated in the form of a salt of mineral acids such as hydrochloric acid, sulfuric acid and phosphoric acid, or of organic acids such as formic acid, acetic acid and oxalic acid.

TAN-749 salts separated in the processes shown above can be converted into free TAN-749 compounds using conventional methods, and said free compounds can be converted into the same salts as above using conventional methods.

Dihydrochlorides of TAN-749A, B, C and D, which were obtained in Example 1 (shown later), possess the following physical and chemical properties:

[1] TAN-749A dihydrochloride

(1) Appearance: Colorless solid

(2) Specific rotation:

$[\alpha]_D^{25}$ - 11° ± 5° (c = 1.06, $H_2O$)

(3) pKa' value: 8.0

(4) Molecular weight: 326 $(M+H)^+$, 348 $(M+Na)^+$

(SI-MS method, M represents the molecular weight of the free compound.)

(5) Molecular formula: $C_{15}H_{27}N_5O_3 \cdot 2HC\ell$

(6) Elemental analysis:

Samples were analyzed after being dried on phosphorous pentoxide under reduced pressure at 40°C for 6 hours. (Calculation was conducted on the condition that one mole of water was contained in the sample.)

|  | Found |  | Calculated |
|---|---|---|---|
| C: | 43.6 ± 2.0 | C: | 43.27 |
| H: | 7.4 ± 1.0 | H: | 7.50 |

(7) Ultraviolet (UV) absorption spectrum (in water):

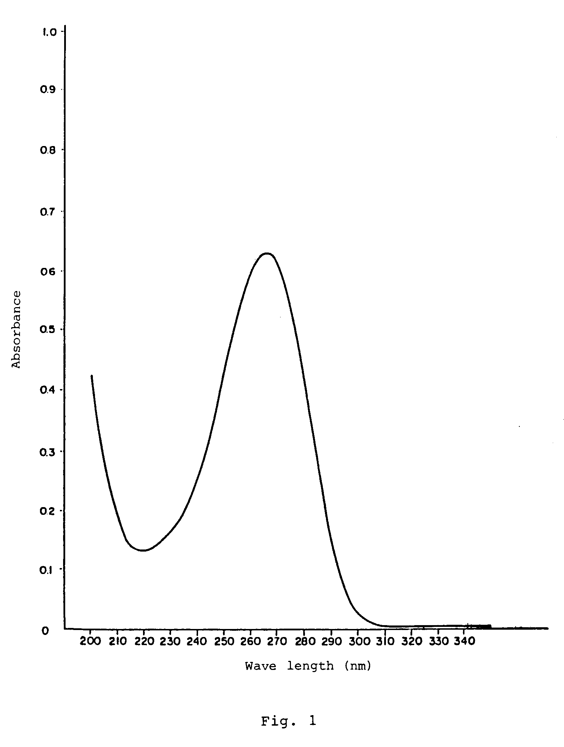

Refer to Fig. 1.

$$\lambda_{\max}\ 266 \pm 3\text{nm}\ (E_{1\text{cm}}^{1\%} = 658 \pm 100)$$

(8) Infrared (IR) absorption spectrum (KBr method):

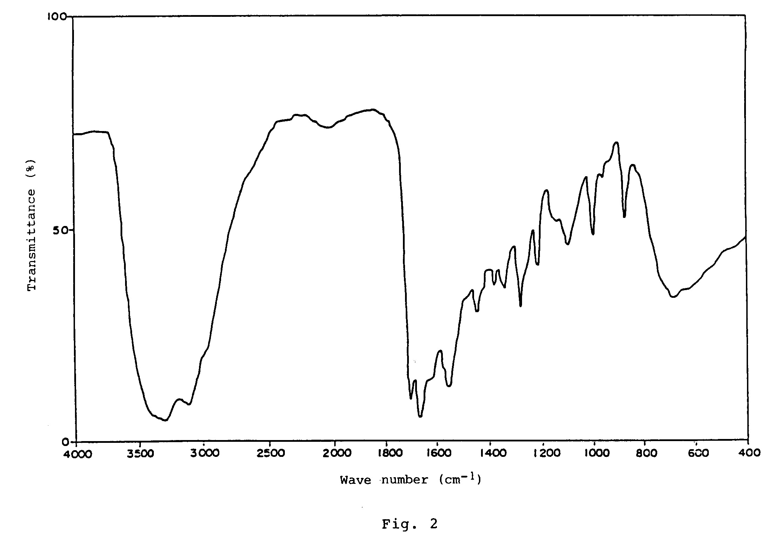

Refer to Fig. 2. Main absorption wavenumbers are as follows:

3400, 3100, 1700, 1670, 1550, 1440, 1380, 1340, 1280, 1220, 1150, 1100, 1000, 960, 870, 680 ($cm^{-1}$)

(9) $^{13}C$ nuclear magnetic resonance (NMR) spectrum:

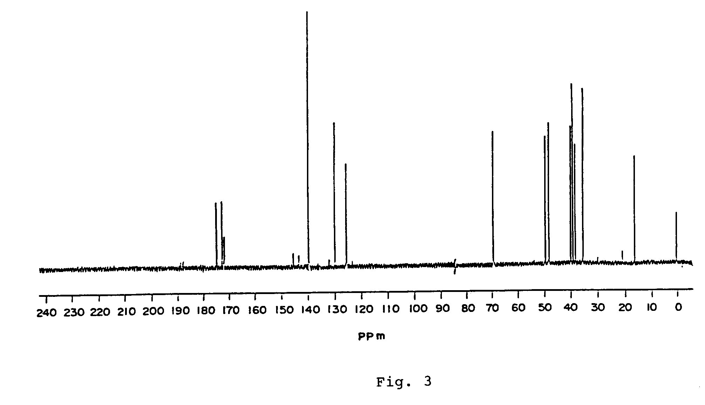

The following signals are measured in deuterium oxide at 100MHz. Refer to Fig. 3.

174.7(s), 172.3(s), 171.7(s), 139.3(d), 139.3(d), 129.6(d), 125.3(d) 69.2(d), 49.2(d), 47.8(t), 39.8(t), 39.1-(t), 38.1(t), 35.2(t), 16.0(q) (ppm)

(Symbols shown above represent signal types, i.e. s: singlet, d: doublet, t: triplet q: quartet.)

(10) High performance liquid chromatography (HPLC);

Retention time : $R_t$ = 4.4 (min)

Column : YMC-PAK A312 (Yamamura Chemical Laboratories)

Mobile phase : 12% methanol/0.01M phosphoric acid solution (pH 3)

Flow rate : 2m$\ell$/min

(11) Color reaction:

Positive: Ehrlich's reaction, Dimethylbenzaldehyde, Potassium permanganate

Negative: Ninhydrin, Greig-Leaback's, Sakaguchi's, Dragendorff's reactions

(12) Solubility:

Soluble : Water, Dimethyl sulfoxide, Methanol

Sparingly soluble : Acetone, Ethyl acetate, Diethyl ether

(13) Acidity or basicity:

Neutral (Free compound is basic.)

[2] TAN-749B dihydrochloride

(1) Appearance: Colorless solid

(2) Specific rotation:

$[\alpha]_D^{25} + 56° \pm 20°$ (c = 1.0, $H_2O$)

(3) pKa' value: 8.05

(4) Molecular weight: 340 (M + H)$^+$, 362 (M + Na)$^+$ (SI-MS method)

(5) Molecular formula: $C_{16}H_{29}N_5O_3 \cdot 2HC\ell$

(6) Elemental analysis

Analysis was conducted under the same conditions with TAN-749A dihydrochloride. (Calculated as 1.5 mole of water was contained in the sample.)

| Found | | Calculated | |
|---|---|---|---|
| C : | 43.62 | C : | 43.94 |
| H : | 7.53 | H : | 7.37 |
| N : | 16.06 | N : | 16.01 |
| O : | | O : | 16.46 |
| C$\ell$: | 16.31 | C$\ell$: | 16.21 (%) |

(7) UV spectrum (in water):

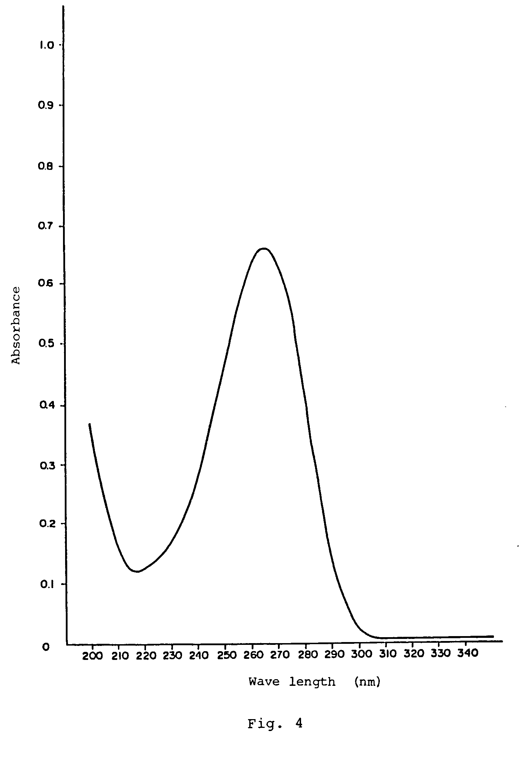

Refer to Fig. 4.

$$\lambda_{max} \ 264 \pm 3nm \ (E_{1cm}^{1\%} = 660 \pm 100)$$

(8) IR spectrum (KBr method):

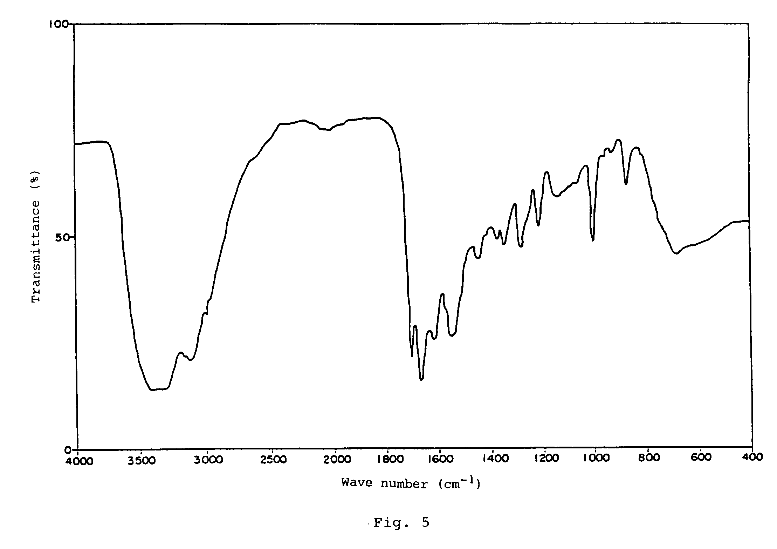

Refer to Fig. 5.

3300, 3100, 1700, 1660, 1610, 1550, 1450, 1420, 1380, 1350, 1280, 1220, 1150, 1070, 1000, 960, 930, 870, 690 (cm$^{-1}$)

(9) $^{13}$C NMR spectrum:

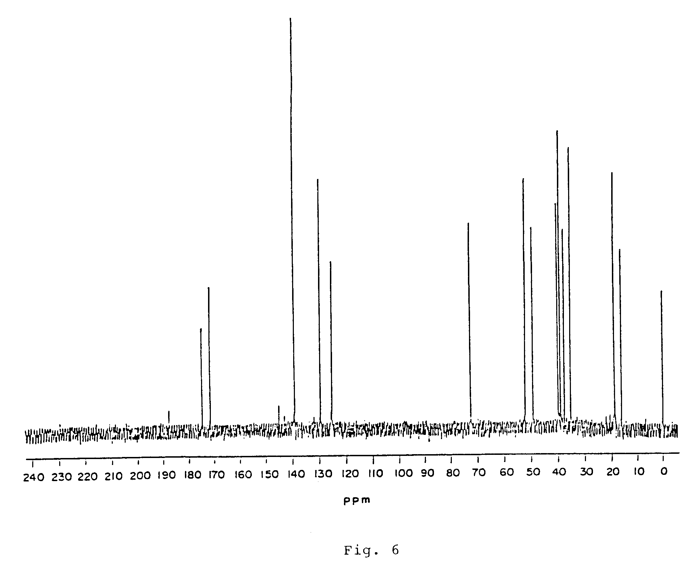

The following signals are measured in deutrium oxide at 100MHz. Refer to Fig. 6.

174.7(s), 171.6(s), 139.3(d), 139.2(d), 129.6(d), 125.5(d), 72.6(d), 52.3(d), 49.3(d), 39.9(t), 39.1(t), 37.5-(t), 35.2(t), 18.8(q), 16.0(q) (ppm)

(10) HPLC:

Retention time: $R_t$ = 7.2 (min)

(Same conditions with TAN-749A)

(11) Color reaction:

Same with TAN-749A dihydrochloride

(12) Solubility:

Same with TAN-749A dihydrochloride

(13) Acidity or basicity:

Neutral (Free compound is basic.)

[3] TAN-749C dihydrochloride

(1) Appearance: Colorless solid

(2) Specific rotation:

$[\alpha]_D^{23} - 11° \pm 5°$ (c = 0.68, $H_2O$)

(3) Molecular weight: 326 (M + H)$^+$, 348 (M + Na)$^+$ (SI-MS method)

(4) Molecular formula: $C_{15}H_{27}N_5O_3 \cdot 2HC\ell$

(5) Elemental analysis:

(Calculated as 0.5 mole of water was contained in the sample.)

| Found | | Calculated | |
|---|---|---|---|
| C : | 44.35 | C : | 44.23 |
| H : | 7.83 | H : | 7.42 |
| N : | 17.28 | N : | 17.19 |
| O : | | O : | 13.75 |
| C$\ell$: | 17.59 | C$\ell$: | 17.41 (%) |

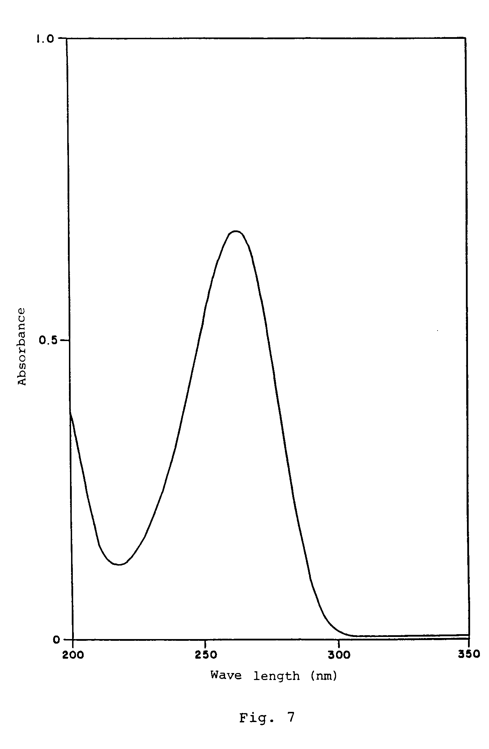

(6) UV spectrum (in water):
Refer to Fig. 7.

$$\lambda_{max} \ 262 \pm 3nm \ (E_{1cm}^{1\%} = 680 \pm 100)$$

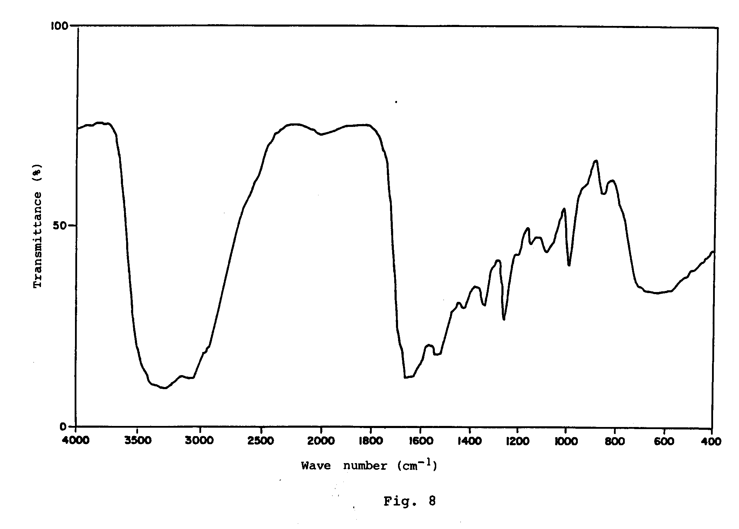

(7) IR spectrum (KBr method):
Refer to Fig. 8.
3250, 3070, 1660, 1630, 1540, 1430, 1340, 1260, 1200, 1150, 1085, 995, 860, 650 (cm$^{-1}$)
(8) $^{13}$C NMR spectrum:
At 100MHz in deuterium oxide
174.7(s), 172.3(s), 171.6(s), 145.1(d), 143.0(d), 132.0(d), 123.1(d), 69.2(d), 49.2(d), 47.7(t), 39.7(t), 39.1-(t), 38.0(t), 35.2(t), 20.6(q) (ppm)
(9) Color reaction:
Same with TAN-749A dihydrochloride
(10) HPLC:

    Retention time    : $R_t = 5.7$ (min) [A:$R_t = 5.3$ (min)]
    Column    : YMC-PAK A312
    Mobile phase    : 30% acetonitrile/0.01M octane sulfonate/0.02M phosphoric acid solution (pH 3.0)
    Flow rate    : 2mℓ/min

(11) Solubility:
Same with TAN-749A dihydrochloride
(12) Acidity or basicity:
Neutral (Free compound is basic.)

[4] TAN-749D dihydrochloride
    (1) Appearance: Colorless solid
    (2) Specific rotation:
    $[\alpha]_D^{24} + 30° \pm 10°$ (c = 0.5, H$_2$O)
    (3) Molecular weight: 340 (M + H)$^+$, 362 (M + Na)$^+$ (SI-MS method)
    (4) Molecular formula: C$_{16}$H$_{29}$N$_5$O$_3$·2HCℓ
    (5) Elemental analysis
    (Calculated as 0.5 mole of water was contained in the sample.)

| Found | | Calculated | |
|---|---|---|---|
| C : | 45.15 | C : | 45.61 |
| H : | 7.98 | H : | 7.65 |
| N : | 16.44 | N : | 16.62 |
| O : | | O : | 13.29 |
| Cℓ: | 16.59 | Cℓ: | 16.83 (%) |

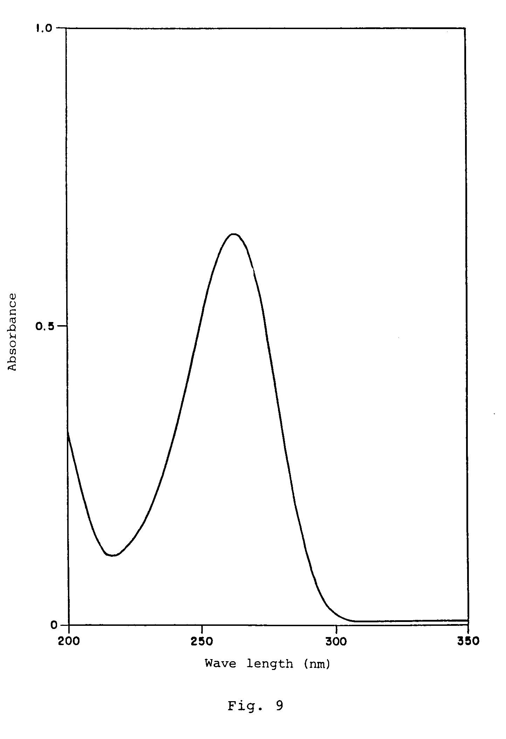

(6) UV spectrum (in water):
Refer to Fig. 9.

$$\lambda_{max} \ 262 \pm 3nm \ (E_{1cm}^{1\%} = 655 \pm 100)$$

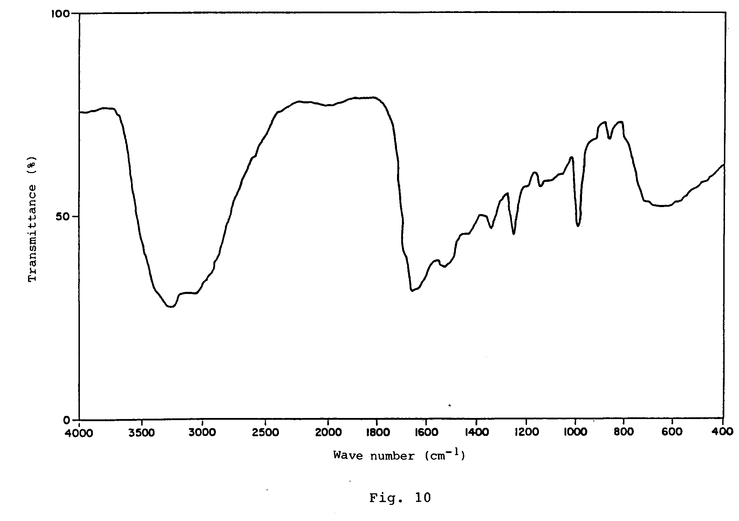

(7) IR spectrum (KBr method):
Refer to Fig. 10.
3250, 3050, 1660, 1635, 1530, 1435, 1345, 1260, 1200, 1150, 995, 860, 650 (cm$^{-1}$)
(8) $^{13}$C NMR spectrum:
At 100MHz in deuterium oxide
174.7(s), 171.7(s), 171.6(s), 145.3(d), 143.0(d), 132.0(d), 123.2(d), 72.5(d), 52.2(d), 49.3(t), 39.9(t), 39.2-(t), 37.5(t), 35.3(t), 20.7(q), 18.8(q) (ppm)
(9) Color reaction:

Same with TAN-749A dihydrochloride
(10) HPLC:
Retention time: $R_t = 6.2$ (min) [B:$R_t = 5.8$ (min)]
(Same conditions with TAN-749C dihydrochloride)
(11) Solubility:
Same with TAN-749A dihydrochloride
(12) Acidity or basicity:
Neutral (Free compound is basic.)

As is evident from the physical and chemical properties described above, TAN-749A and C, and TAN-749B and D, are stereoisomers of each other, respectively.

The biological characteristics of TAN-749 are described hereinafter.

Tables 2 and 3 show the antibacterial spectra of TAN-749A, B, C and D (dihydrochlorides) against various microorganisms.

### Table 2

| Test Organism | Minimal Inhibitory Concentration (Note 1) ($\mu$g/m$\ell$) | | | |
|---|---|---|---|---|
| | A | B | C | D |
| Staphylococcus aureus FDA 209P | 50 | 12.5 | >100 | 50 |
| Escherichia coli NIHJ JC2 | >100 | >100 | >100 | >100 |
| Citrobacter freundii IFO 12681 | ≧100 | ≧100 | >100 | >100 |
| Klebsiella pneumoniae IFO 3317 | >100 | 100 | >100 | >100 |
| Proteus vulgaris IFO 3988 | 100 | 25 | 100 | 100 |
| Proteus morganii IFO 3168 | >100 | 100 | >100 | >100 |
| Pseudomonas aeruginosa IFO 3080 | 25 | 50 | 50 | 100 |
| Alcaligenes faecalis IFO 13111 | 3.13 | 6.25 | 12.5 | 6.25 |
| Acinetobacter calcoaceticus IFO 13006 | 25 | 50 | >100 | >100 |

(Note 1) Medium composition

| | |
|---|---|
| Bacto-Antibiotic Medium 3 (Difco Laboratories, USA) : | 17.5g |
| Bacto-yeast extract (Difco Laboratories, USA) : | 5.0g |
| Bacto-agar (Difco Laboratories, USA) : | 20g |
| Distilled water (pH unadjusted) : | 1,000m$\ell$ |
| Inoculum size : | a loopful of approx. $10^6$ CFU/m$\ell$ |

11

EP 0 206 068 B1

Table 3

| Test Organism | Medium (Note 2) | Minimal Inhibitory Concentration (Note 1) ($\mu$g/m$\ell$) | | | |
|---|---|---|---|---|---|
| | | A | B | C | D |
| Staphylococcus aureus 308A-1 | TSA | 12.5 | 3.13 | >100 | 25 |
| Escherichia coli T7 | TSA | 50 | 12.5 | >100 | 50 |
| Staphylococcus aureus FDA209P | B-TSA | 12.5 | 3.13 | >100 | 25 |
| Streptococcus pyogenes E-14 | B-TSA | 3.13 | 6.25 | 100 | 25 |
| Pseudomonas aeruginosa P9 | B-TSA | 100 | 50 | 100 | 100 |
| (Note 1) Determined by the agar dilution method. Inoculum size was a loopful of $10^8$ CFU/m$\ell$. (Note 2) TSA (Tripticase Soy Agar; Baltimore Biological Laboratories, USA), B-TSA; 10% horse serum/TSA | | | | | |

Table 4 shows the antibacterial activities of TAN-749A dihydrochloride against clinically isolated Staphylococcus aureus strains.

Table 4

| Strain | Resistance Type | Minimal Inhibitory Concentration (Note 1) ($\mu$g/m$\ell$) |
|---|---|---|
| 1840 S | None | 12.5 |
| 1840-2 | Penicillin G | 12.5 |
| TN 2613 | Methicillin | 6.25 |
| TN 2648 | Methicillin | 3.13 |
| TN 2687 | Macrolide | 6.25 |
| TN 2684 | Macrolide | 6.25 |
| TN 2688 | Macrolide | 6.25 |
| (Note 1) Determined by the agar dilution method. Medium: Mueller Hinton medium (Difco, USA) Inoculum size: a loopful of $10^6$ CFU/m$\ell$ | | |

Table 5 shows the therapeutic effects of TAN-749A, B C and D (dihydrochlorides) to infectious diseases in mice.

Table 5

| Bacteria Infected Intraperitoneally | Route of Administration | $ED_{50}$ (mg/kg) | | | |
|---|---|---|---|---|---|
| | | A | B | C | D |
| Escherichia coli 0-111 | Subcutaneous | 67.2 | 27.3 | 50 | |
| Escherichia coli T7 | Subcutaneous | 50.8 | | | |
| Pseudomonas aeruginosa P-9 | Subcutaneous | 31.0 | 61.4 | | |
| Staphylococcus aureus 308A-1 | Subcutaneous | 1.31 | 0.351 | 12.5 | <6.25 |
| Staphylococcus aureus 308A-1 | Oral | 17.7 | 16.2 | | |

Table 6 shows the preliminary acute toxicities of TAN-749A and B (dihydrochlorides) in mice.

12

Table 6

| Route | LD$_{50}$ (mg/kg) | |
|---|---|---|
| | A | B |
| Subcutaneous | Approx. 500 | 400~800 |
| Oral | 2000~4000 | |

As described clearly from these data, TAN-749 and its salts have antibacterial activity against both gram-positive and gram-negative bacteria and their acute toxicities in mammals are low. Moreover, they are effective to various types of drug-resistant bacteria and do not show cross resistance.

Therefore, TAN-749 and its salts can be used in the therapeutics of bacterial infections in mammals (mice, rats, rabbits, dogs, humans, etc.).

TAN-749 or its salts can be used as therapeutic drugs in the following manners; TAN-749 or its salts, is mixed with pharmacologically allowable carriers. For instance, in the production of oral preparations, there may be employed suitable amounts of binders (e.g. hydroxypropyl-cellulose, hydroxypropylmethyl-cellulose, macrogol, etc.), disintegrators (e.g. starch, carboxymethyl-cellulose calcium, etc.), excipients (e.g. lactose, starch, etc.), lubricants (e.g. magnesium stearate, talc, etc.) and so on.

In the production of parenteral therapeutics e.g. injections, there may be employed isotonicating agents (e.g. glucose, D-sorbitol, D-mannitol, sodium chloride, etc.), preservatives (e.g. benzyl alcohol, chlorobutanol, methyl p-hydroxybenzoate, propyl p-phydroxybenzoate, etc.), buffers (e.g. phophate buffer, sodium acetate buffer, etc.) and so on. They are given parenterally to said mammals via subcutaneous or intramuscular injection at a dose of approx. 1 to 50mg/kg/day, preferably approx. 5 to 20mg/kg/day. When TAN-749 or its salt is given orally, they can be given in the form of capsules at a TAN-749 dose of approx. 1 to 100mg/kg/day; it is recommended that the dose is between approx. 5 and 50mg/kg/day.

TAN-749 or its salts can be used as bactericides. For example, hands, legs, faces, ears, etc. can be sterilized and disinfected by applying it over these portions as a liquid prepared by disslolving TAN-749 or its salts in distilled water at a concentration approx. 0.01 to 0.1 w/v% or an ointment containing approx. 0.2 to 20mg, preferably approx. 1 to 10mg, of TAN-749 per gram.

The present invented products, TAN-749A, B, C and D, are new antibiotics produced by bacteria and are effective against gram-positive and gram-negative bacteria including drug-resistant ones; therefore, they can be used favorably as clinical drugs, e.g. therapeutic drugs for bacterial infection.

The present invention is described in more detail with some examples of its preferred embodiments hereinafter. Medium composition contents are expressed in weight/volume % unless otherwise stated.

Example 1:

Pseudomonas fluorescens YK-437 (IFO 14446, FERM BP-1005) grow on an enriched agar slant medium was inoculated into a 2ℓ Sakaguchi flask containing 500mℓ of a medium prepared by adding 0.5% precipitating calcium carbonate to an aqueous solution (pH 7.0) containing 2% glucose, 3% soluble starch, 1% soybean flour, 0.3% corn steep liquor, 0.5% Polypepton (Daigo Nutritive Chemicals, Japan) and 0.3% sodium chloride, after which it was subjected to reciprocal shaking culture at 24°C for 48 hours. The entire quantity of the resulting culture broth was then inoculated into a 50ℓ fermentor containing 30ℓ of a medium prepared by adding 0.05% Actcol (Takeda Chemical Industries, Japan), an antifoaming agent, to said medium, and cultured at 24°C, a 30ℓ/min aeration rate and 200 rpm/min for 48 hours. Six liters of the resulting culture broth was then inoculated into a 200ℓ fermentor containing 120ℓ of a medium containing 3% glycerol, 0.1% glucose, 0.5% Polypepton, 0.5% meat extract (Wako Pure Chemical Industries, Japan), 0.5% sodium chloride, 0.05% sodium thiosulfate, 2 ppm cobalt chloride and 0.05% Actcol after which it was incubated at 24°C for 66 hours under the aeration of 120ℓ/min and agitaion of 170 rpm.

The resulting culture broth (105ℓ), after being adjusted to pH 6.5 with 2N hydrochloric acid, was added to a Hyflo super Cel (Johns Manville Product, USA) and subjected to filtration and water washing, yielding a filtrate (102ℓ). The resulting filtrate, after adjusting to pH 6.5, was passed through a column packed with IRC-50 (Na$^+$ type, 2ℓ). The column, after washing with water, was subjected to elution with a 2M saline solution (500ℓ). The resulting eluate was passed through a column packed with activated charcoal (2ℓ), washed with water, and then subjected to elution using an 8% isobutanol slash water solution (15ℓ) as an eluent. The resulting eluate, after adjusting to pH 6.2, was concentrated to 2ℓ and then passed through a column packed with CM-Sephadex C-25 (Na$^+$ type, 0.5ℓ). Active fractions were then eluted using a 0.1M

saline solution (20ℓ) as an eluent.

A TAN-749B fraction appeared in the former part of the chromatogram and a TAN-749A fraction appeared in the latter part of the chromatogram.

Each resulting fraction was subjected to chromatography using activated charcoal (1.0ℓ or 4.0ℓ) and after desalination, it was concentrated and lyophilized, yielding a crude TAN-749B (4.0g) or a crude TAN-749A (8.9g).

Crude product B (4.0g) was then subjected to reversed-phase HPLC for separation [Carrier: YMC-PAK S-30 (Yamamura Chemical Laboratories, Japan); Mobil phase: 8% methanol/0.02M phosphoric acid solution (pH 3)], yielding an active fraction. The resulting active fractions were jected to column chromatography using CM-Sephadex C-25 (Na$^+$ type, 0.25ℓ) and then subjected to column chromatography using activated charcoal (0.3ℓ), yielding a purified fraction. The resulting fraction was then concentrated and lyophilized, yielding TAN-749B dihydrochloride (0.66g) as a white powder. Crude product A (8.9g) was treated with the same processes, yielding TAN-749A dihydrochloride as a white powder (4.7g).

Example 2:

Pseudomonas fluorescens YK-437 (IFO 14446, FERM BP-1005) grown on an enriched agar slant medium was inoculated into a 2ℓ Sakaguchi flask containing 500mℓ of a medium prepared by adding 0.5% precipitating calcium carbonate to an aqueous solution (pH 7.0) containing 2% glucose, 3% soluble starch, 1% soybean flour, 0.3% corn steep liquor, 0.5% Polypepton, and 0.3% sodium chloride, after which it was subjected to reciprocal shaking culture at 24°C for 48 hours. The entire quantity of the resulting culture broth was inoculated into a 200ℓ fermentor containing 120ℓ of a medium prepared by adding 0.05% Actcol to said medium, and then incubated at 24°C, a 120ℓ/min aeration rate and 180 rpm/min, for 48 hours. Fifty liters of the resulting culture broth was inoculated into a 2,000ℓ fermentor containing 1,200ℓ of medium contining 3% glycerol, 0.1% glucose, 0.5% Polypepton (Daigo Nutritive Chemicals), 0.5% meat extract (Wako Pure Chemicals, Japan), 0.5% sodium chloride, 0.05% sodium thiosulfate, 2 ppm cobalt chloride and 0.05% Actcol after which it was incubated at 24°C for 66 hours under the aeration of 1,200ℓ/min and agitation of 150 rpm.

The culture broth obtained (1,150ℓ), after adjusting to pH 6.5, was added to a Hyflo Super Cel (Johns Manville Product, USA) and then subjected to filtration and water washing, yielding a filtrate (1,220ℓ). The resulting filtrate, after adjusting to pH 6.2, was passed through a column packed with IRC-50 (Na$^+$ type, 20ℓ). The column, after washing with water, was subjected to elution using a 0.5N hydrochloric acid solution (200ℓ) as an eluent. The resulting eluate, after adjusting to pH 5.6, was passed through a column packed with Diaion SP-207 (20ℓ) and then subjected to elution with water (120ℓ). The resulting eluate was concentrated to 2ℓ, the concentrate was passed through a column packed with CG-50 (NH$_4$$^+$ type, 3ℓ). Active fractions were then eluted using a 0.40~0.6M saline slution (40ℓ) as a eluent.

TAN-749A, B, C and D fractions appeared in the former part of the chromatogram and a TAN-749A fraction appeared in the latter part.

Each resulting fraction was subjected to chromatography using activated charcoal (1.2ℓ or 2.0ℓ) as the packing, and eluted with an 8% isobutanol water solution (4ℓ or 10ℓ). The fraction containing TAN-749A alone was concentrated and lyophilized, yielding TAN-749A (47.5g).

As for the fraction containing TAN-749A, B, C and D, 3 lots (corresponds to 3,450ℓ of the initial culture broth ) of such fractions obtained in the same processes, were concentrated. The resulting concentrate (2ℓ) was then subjected to column chromatography using CG-50 (NH$_4$$^+$ type, 3ℓ) as the packing. The column, after washing with a 0.2M saline solution, was subjected to elution using a 0.5~0.8M saline solution (40ℓ) as an eluent. A fraction containing TAN-749B and D appeared in the former part of the chromatogram and one containing TAN-749A and C appeared in the latter part. The fraction containing TAN-749A and C was subjected to chromatography using activated charcoal as the packing and desalted. The resulting eluate was concentrated and lyophilized, yielding a TAN-749A powder (20g) containing a small amount of TAN-749C.

The fraction containing TAN-749B and D was subjected to chromatography using activated charcoal as the packing and desalted. The resulting eluate was subjected to column chromatography using CM-Sephadex C-25 (Na$^+$ type 1ℓ) as the packing and elution using a 0.2M saline solution as an eluent. The resulting eluate was concentrated; and the resulting concentrate was subjected to reversed-phase HPLC for separation [Carrier: ODS, YMC-PAK S-30; Mobile phase: 5% methanol/0.02M phosphoric acid solution (pH 3.0)], yielding two fractions, i.e. a fraction containing TAN-749B alone and one containing TAN-749B and D. The fraction containing TAN-749B alone was subjected to chromatography using CM-Sephadex and then activated charcoal as packings, yieldings TAN-749B (3.05g). The fraction containing TAN-749B and D was concentrated and then subjected to HPLC again. The resulting fraction containing TAN-749D alone was then concentrated. The resulting concentrate was passed through a column packed with IRA-402 (Cℓ⁻ type, 10mℓ) and the column was washed with water. The effluent was subjected to chromatography using activated charcoal for desalination, yielding TAN-749D (15.5mg).

The TAN-749A powder (3g) containing a small amount of TAN-749C, obtained above, was subjected to chromatography using CM-Sephadex, CG-50 and then activated charcoal as packings to increase the ratio of TAN-749C content. The resulting powder with a high TAN-749C content was purified via two repetitions of HPLC under the conditions shown above, yielding TAN-749C (20.2mg).

Example 3:

| Capsule | |
|---|---|
| (1) TAN-749A | 300mg |
| (2) Lactose | 28mg |
| (3) Corn starch | 58mg |
| (4) Hydroxy propyl cellulose | 12mg |
| (5) Magnesium stearate | 2mg |
| | 400mg/capsule |

The above ingredients (1), (2), (3) and (4) are mixed and granulated by the conventional method. To the granules is added the ingredient (5). The mixture is packed into a gelatin capsule No. 1 (according to the Pharmacopoeia of Japan, Tenth Edition).

Example 4:

20g of TAN-749B is dissolved into one liter of distilled water. After adding and solving 50g of mannitol, each 2mℓ of the solution is poured into an ample after filtration for sterilization. This is lyophilized, and sealed to prepare amples.

When it is used, the said ample is opened and dissolved into 2mℓ of physiological saline to prepare a subcutaneous or intramuscular injection.

## Claims

## Claims for the following Contracting States : BE, CH, LI, DE, FR, GB, IT, LU, NL, SE

1. Antibiotics TAN-749A, B, C and D which possess the following physical and chemical properties as dihydrochlorides, or their salts.

   TAN-749A
   (1) Appearance: Colorless solid
   (2) Molecular formula: $C_{15}H_{27}N_5O_3 \cdot 2HC\ell$
   (3) Ultraviolet (UV) absorption spectrum (in water):

$$\lambda_{max}\ 266 \pm 3nm\ (E_{1cm}^{1\%} = 658 \pm 100)$$

   (4) Infrared (IR) absorption spectrum (KBr method):
   Main wavenumbers are as follows:
   3400, 3100, 1700, 1670, 1550, 1440, 1380, 1340, 1280, 1220, 1150, 1100, 1000, 960, 870, 680 $(cm^{-1})$
   (5) $^{13}C$ nuclear magnetic resonance (NMR) spectrum:
   The following signals are measured in deuteriun oxide at 100 MHz:
   174.7(s), 172.3 (s), 171.7(s), 139.3(d), 139.3(d), 129.6(d), 125.3(d), 69.2(d), 49.2(d), 47.8(t), 39.8(t), 39.1(t), 38.1(t), 35.2(t), 16.0(q) (ppm)
   (6) Color reaction:
       Positive:     Ehrlich's reaction, Dimethylbenzaldehyde, Potassium permanganate
       Negative:    Ninhydrin, Greig-Leaback's, Sakaguchi's, Dragendorff's reactions
   TAN-749B

(1) Appearance: Colorless solid
(2) Molecular formula: $C_{16}H_{29}N_5O_3 \cdot 2HC\ell$
(3) UV spectrum (in water):

$$\lambda_{max} \; 264 \pm 3nm \; (E_{1cm}^{1\%} = 660 \pm 100)$$

(4) IR spectrum (KBr method):
3300, 3100, 1700, 1660, 1610, 1550, 1450, 1420, 1380, 1350, 1280, 1220, 1150, 1070, 1000, 960, 930, 870, 690 (cm$^{-1}$)
(5) $^{13}$C NMR spectrum:
The following signals are measured in deuterium oxide at 100 MHz:
174.7(s), 171.6(s), 139.3(d), 139.2(d), 129.6(d), 125.5(d), 72.6(d), 52.3(d), 49.3(d), 39.9(t), 39.1(t), 37.5(t), 35.2(t), 18.8(q) 16.0(q) (ppm)
(6) Color reaction:
    Positive:     Ehrlich's reaction, Dimethylbenzaldehyde, Potassium permanganate
    Negative:    Ninhydrin, Greig-Leaback's, Sakaguchi's, Dragendorff's reactions

TAN-749C
(1) Appearance: Colorless solid
(2) Molecular formula: $C_{15}H_{27}N_5O_3 \cdot 2HC\ell$
(3) UV spectrum (in water):

$$\lambda_{max} \; 262 \pm 3nm \; (E_{1cm}^{1\%} = 680 \pm 100)$$

(4) IR spectrum (KBr method):
3250, 3070, 1660, 1630, 1540, 1430, 1340, 1260, 1200, 1150, 1085, 995, 860, 650 (cm$^{-1}$)
(5) $^{13}$C NMR spectrum:
The following signals are measured in deuterium oxide at 100 MHz:
174.7(s), 172.3(s), 171.6(s), 145.1(d), 143.0(d), 132.0(d), 123.1(d), 69.2(d), 49.2(d), 47.7(t), 39.7(t), 39.1(t), 38.0(t), 35.2(t), 20.6(q) (ppm)
(6) Color reaction:
    Positive:     Ehrlich's reaction, Dimethylbenzaldehyde, Potassium permanganate
    Negative:    Ninhydrin, Greig-Leaback's, Sakaguchi's, Dragendorff's reactions

TAN-749D
(1) Appearance: Colorless solid
(2) Molecular formula: $C_{16}H_{29}N_5O_3 \cdot 2HC\ell$
(3) UV spectrum (in water):

$$\lambda_{max} \; 262 \pm 3nm \; (E_{1cm}^{1\%} = 655 \pm 100)$$

(4) IR spectrum (KBr method):
3250, 3050, 1660, 1635, 1530, 1435, 1345, 1260, 1200, 1150, 995, 860, 650 (cm$^{-1}$)
(5) $^{13}$C NMR spectrum:
The following signals are measured in deuterium oxide at 100 MHz:
174.7(s), 171.7(s), 171.6(s), 145.3(d), 143.0(d), 132.0(d), 123.2(d), 72.5(d), 52.2(d), 49.3.(d), 39.9(t), 39.2(t), 37.5(t), 35.3(t), 20.7(q), 18.8(q) (ppm)
(6) Color reaction:
    Positive:     Ehrlich's reaction, Dimethylbenzaldehyde, Potassium permanganate
    Negative:    Ninhydrin, Greig-Leaback's, Sakaguchi's, Dragendorff's reactions

2.   A compound as claimed in Claim 1, wherein the compound is dihydrochloride of antibiotic TAN-749A, B, C or D.

3.   A method for producing antibiotics TAN-749A, B, C and D as defined in claim 1 or their salts, which comprises incubating a microbe belonging to the genus Pseudomonas capable of producing one or more of antibiotics TAN-749A, B, C and D in a medium to produce and accumulate one or more of antibiotics TAN-749A, B, C and D in the culture broth and then to recover it (them).

4. A method as claimed in Claim 3, wherein the microbe is Pseudomonas fluorescens.

5. A method as claimed in Claim 3, wherein the microbe is Pseudomonas fluorescens YK-437 (FERM BP-1005).

6. A pharmaceutical composition for treating bacterial infections, which contains an effective amount of one or more of antibiotics TAN-749A, B, C and D as defined in claim 1, and a pharmacologically allowable carrier.

7. A biologically pure culture of Pseudomonas fluorescens YK-437 (FERM BP-1005), and mutants and variants thereof capable of producing one or more of antibiotics TAN-749 A, B, C and D as defined in claim 1.

**Claims for the following Contracting State : AT**

1. A method for producing antibiotics TAN-749A, B, C and D which possess the following physical and chemical properties as dihydrochlorides, or their salts.

TAN-749A

(1) Appearance: Colorless solid

(2) Molecular formula: $C_{15}H_{27}N_5O_3 \cdot 2HC\ell$

(3) Ultraviolet (UV) absorption spectrum (in water):

$$\lambda_{max} \ 266 \pm 3nm \ (E_{1cm}^{1\%} = 658 \pm 100)$$

(4) Infrared (IR) absorption spectrum (KBr method):

Main wavenumbers are as follows:

3400, 3100, 1700, 1670, 1550, 1440, 1380, 1340, 1280, 1220, 1150, 1100, 1000, 960, 870, 680 $(cm^{-1})$

(5) $^{13}C$ nuclear magnetic resonance (NMR) spectrum:

The following signals are measured in deuterium oxide at 100 MHz:

174.7(s), 172.3(s), 171.7(s), 139.3(d), 139.3(d), 129.6(d), 125.3(d), 69.2(d), 49.2(d), 47.8(t), 39.8(t), 39.1(t), 38.1(t), 35.2(t), 16.0(q) (ppm)

(6) Color reaction:

    Positive:      Ehrlich's reaction, Dimethylbenzaldehyde, Potassium permanganate

    Negative:    Ninhydrin, Greig-Leaback's, Sakaguchi's, Dragendorff's reactions

TAN-749B

(1) Appearance: Colorless solid

(2) Molecular formula: $C_{16}H_{29}N_5O_3 \cdot 2HC\ell$

(3) UV spectrum (in water):

$$\lambda_{max} \ 264 \pm 3nm \ (E_{1cm}^{1\%} = 660 \pm 100)$$

(4) IR spectrum (KBr method):

3300, 3100, 1700, 1660, 1610, 1550, 1450, 1420, 1380, 1350, 1280, 1220, 1150, 1070, 1000, 960, 930, 870, 690 $(cm^{-1})$

(5) $^{13}C$ NMR spectrum:

The following signals are measured in deuteriun oxide at 100 MHz:

174.7(s), 171.6(s), 139.3(d), 139.2(d), 129.6(d), 125.5(d), 72.6(d), 52.3(d), 49.3(d), 39.9(t), 39.1(t), 37.5(t), 35.2(t), 18.8(q) 16.0(q) (ppm)

(6) Color reaction:

    Positive:      Ehrlich's reaction, Dimethylbenzaldehyde, Potassium permanganate

    Negative:    Ninhydrin, Greig-Leaback's, Sakaguchi's, Dragendorff's reactions

TAN-749C

(1) Appearance: Colorless solid

(2) Molecular formula: $C_{15}H_{27}N_5O_3 \cdot 2HC\ell$

(3) UV spectrum (in water):

$$\lambda_{max} \ 262 \pm 3nm \ (E^{1\%}_{1cm} = 680 \pm 100)$$

(4) IR spectrum (KBr method):

3250, 3070, 1660, 1630, 1540, 1430, 1340, 1260, 1200, 1150, 1085, 995, 860, 650 $(cm^{-1})$

(5) $^{13}C$ NMR spectrum:

The following signals are measured in deuterium oxide at 100 MHz:

174.7(s), 172.3(s), 171.6(s), 145.1(d), 143.0(d), 132.0(d), 123.1(d), 69.2(d), 49.2(d), 47.7(t), 39.7(t), 39.1(t), 38.0(t), 35.2(t), 20.6(q) (ppm)

(6) Color reaction:

    Positive:    Ehrlich's reaction, Dimethylbenzaldehyde, Potassium permanganate

    Negative:    Ninhydrin, Greig-Leaback's, Sakaguchi's, Dragendorff's reactions

TAN-749D

(1) Appearance: Colorless solid

(2) Molecular formula: $C_{16}H_{29}N_5O_3 \cdot 2HC\ell$

(3) UV spectrum (in water):

$$\lambda_{max} \ 262 \pm 3nm \ (E^{1\%}_{1cm} = 655 \pm 100)$$

(4) IR spectrum (KBr method):

3250, 3050, 1660, 1635, 1530, 1435, 1345, 1260, 1200, 1150, 995, 860, 650 $(cm^{-1})$

(5) $^{13}C$ NMR spectrum:

The following signals are measured in deuterium oxide at 100 MHz:

174.7(s), 171.7(s), 171.6(s), 145.3(d), 143.0(d), 132.0(d), 123.2(d), 72.5(d), 52.2(d), 49.3(d), 39.9(t), 39.2(t), 37.5(t), 35.3(t), 20.7(q), 18.8(q) (ppm)

(6) Color reaction:

    Positive:    Ehrlich's reaction, Dimethylbenzaldehyde, Potassium permanganate

    Negative:    Ninhydrin, Greig-Leaback's, Sakaguchi's, Dragendorff's reactions

which method comprises incubating a microbe belonging to the genus Pseudomonas capable of producing one or more of antibiotics TAN-749, B, C and D in a medium to produce and accumulate one or more of antibiotics TAN-749A, B, C and D in the culture broth and then to recover it (them).

2. A method as claimed in the Claim 1, wherein the microbe is Pseudomonas fluorescens.

3. A method as claimed in Claim 1, wherein the microbe is Pseudomonas fluorescens YK-437 (FERM BP-1005).

**Patentansprüche**

**Patentansprüche für folgende Vertragsstaaten : BE, CH, LI, DE, FR, GB, IT, LU, NL, SE**

1. Antibiotika TAN-749A, B, C und D, welche als Dihydrochloride die folgenden physikalischen und chemischen Eigenschaften besitzen, oder deren Salze :

TAN-749A

(1) Erscheinungsbild: farbloser Feststoff

(2) Molekularformel: $C_{15}H_{27}N_5O_3 {}^*2HCl$

(3) UV- (Ultraviolett) Absorptionsspektrum (in Wasser):

$$\lambda_{max} \ 266 \pm 3 \ nm \ (E^{1\%}_{1cm} = 658 \pm 100)$$

(4) Infrarot- (IR) Absorptionsspektrum (KBr-Methode):

Die Hauptwellenzahlen sind wie folgt:

3400, 3100, 1700, 1670, 1550, 1440, 1380, 1340, 1280, 1220, 1150, 1100, 1000, 960, 870, 680 (cm$^{-1}$)

(5) $^{13}$C-Kernmag netisches Resonanz- (NMR) Spektrum:

Die folgenden Signale sind in Deuteriumoxid bei 100 MHz gemessen:

174,7 (s), 172,3 (s), 171,7 (s), 139,3 (d), 139,3 (d), 129,6 (d), 125,3 (d), 69,2 (d), 49,2 (d), 47,8 (t), 39,8 (t), 39,1 (t), 38,1 (t), 35,2 (t), 16,0 (q) (ppm)

(6) Farbreaktion:

    Positiv:     Ehrlich-Reaktion, Dimethylbenzaldehyd, Kaliumpermanganat

    Negativ:    Ninhydrin, Greig-Leaback-, Sakaguchi-, Dragendorff-Reaktion

TAN-749B

(1) Erscheinungsbild: farbloser Feststoff

(2) Molekularformel: $C_{16}H_{29}N_5O_3$*2HCl

(3) UV-Spektrum (in Wasser):

$$\lambda_{max}\ 264 \pm 3\ nm\ (E^{1\%}_{1cm} = 660 \pm 100)$$

(4) IR-Spektrum (KBr-Methode):

3300, 3100, 1700, 1660, 1610, 1550, 1450, 1420, 1380, 1350, 1280, 1220, 1150, 1070, 1000, 960, 930, 870, 690 (cm$^{-}$1)

(5) $^{13}$C-NMR-Spektrum:

Die folgenden Signale sind in Deuteriumoxid bei 100 MHz gemessen:

174,7 (s), 171,6 (s), 139,3 (d), 139,2 (d), 129,6 (d), 125,5 (d), 72,6 (d), 52,3 (d), 49,3 (d), 39,9 (t), 39,1 (t), 37,5 (t), 35,2 (t), 18,8 (q), 16,0 (q) (ppm)

(6) Farbreaktion:

    Positiv:     Ehrlich-Reaktion, Dimethylbenzaldehyd, Kaliumpermanganat

    Negativ:    Ninhydrin, Greig-Leaback-, Sakaguchi-, Dragendorff-Reaktion

TAN-749C

(1) Erscheinungsbild: farbloser Feststoff

(2) Molekularformel: $C_{15}H_{27}N_5O_3$*2HCl

(3) UV-Spektrum (in Wasser):

$$\lambda_{max}\ 262 \pm 3\ nm\ (E^{1\%}_{1cm} = 680 \pm 100)$$

(4) IR-Spektrum (KBr-Methode):

3250, 3070, 1660, 1630, 1540, 1430, 1340, 1260, 1200, 1150, 1085, 995, 860, 650 (cm$^{-1}$)

(5) $^{13}$C-NMR-Spektrum:

Die folgenden Signale sind in Deuteriumoxid bei 100 MHz gemessen:

174,7 (s), 172,3 (s), 171,6 (s), 145,1 (d), 143,0 (d), 132,0 (d), 123,1 (d), 69,2 (d), 49,2 (d), 47,7 (t), 39,7 (t), 39,1 (t), 38,0 (t), 35,2 (t), 20,6 (q) (ppm)

(6) Farbreaktion:

    Positiv:     Ehrlich-Reaktion, Dimethylbenzaldehyd, Kaliumpermanganat

    Negativ:    Ninhydrin, Greig-Leaback-, Sakaguchi-, Dragendorff-Reaktion

TAN-749D

(1) Erscheinungsbild: farbloser Feststoff

(2) Molekularformel: $C_{16}H_{29}N_5O_3$*2HCl

(3) UV-Spektrum (in Wasser):

$$\lambda_{max}\ 262 \pm 3\ nm\ (E^{1\%}_{1cm} = 655 \pm 100)$$

(4) IR-Spektrum (KBr-Methode):

3250, 3050, 1660, 1635, 1530, 1435, 1345, 1260, 1200, 1150, 995, 860, 650 (cm$^{-1}$)

(5) $^{13}$C-NMR-Spektrum:

Die folgenden Signale sind in Deuteriumoxid bei 100 MHz gemessen:

174,7 (s), 171,7 (s), 171,6 (s), 145,3 (d), 143,0 (d), 132,0 (d), 123,2 (d), 72,5 (d), 52,2 (d), 49,3 (d), 39,9 (t), 39,2 (t), 37,5 (t), 35,3 (t), 20,7 (q), 18,8 (q) (ppm)

(6) Farbreaktion:

    Positiv:      Ehrlich-Reaktion, Dimethylbenzaldehyd, Kaliumpermanganat

    Negativ:     Ninhydrin, Greig-Leabacks, Sakaguchis, Dragendorffs Reaktionen

**2.** Verbindung wie in Anspruch 1 beansprucht, wobei die Verbindung ein Dihydrochlorid des Antibiotikums TAN-749A, B, C oder D ist.

**3.** Verfahren zur Herstellung der wie in Anspruch 1 definierten Antibiotika TAN-749A, B, C oder D oder ihrer Salze, welches das Inkubieren eines zur Gattung Pseudomonas gehörenden, zur Produktion eines oder mehrerer der Antibiotika TAN-749A, B, C oder D befähigten Mikroorganismus in einem Medium unter Erhalt und Anreicherung eines oder mehrerer der Antibiotika TAN-749A, B, C oder D in der Kulturbrühe und darauf seine (ihre) Gewinnung umfaßt.

**4.** Verfahren wie in Anspruch 3 beansprucht, wobei der Mikroorganismus Pseudomonas fluorescens ist.

**5.** Verfahren wie in Anspruch 3 beansprucht, wobei der Mikroorganismus Pseudomonas fluorescens YK-437 (FERM BP-1005) ist.

**6.** Pharmazeutische Zusammensetzung zur Behandlung bakterieller Infektionen, welche eine wirksame Menge eines oder mehrerer wie in Anspruch 1 definierter Antibiotika TAN-749A, B, C oder D und einen pharmakologisch zulässigen Träger enthält.

**7.** Biologisch reine Kultur von Pseudomonas fluorescens YK-437 (FERM BP-1005) und dessen Mutanten und Varianten, welche befähigt sind, einen oder mehrere wie in Anspruch 1 definierte Antibiotika TAN-749A, B, C oder D zu produzieren.

**Patentansprüche für folgenden Vertragsstaat : AT**

**1.** Verfahren zur Herstellung der Antibiotika TAN-749A, B, C und D, welche als Dihydrochloride die folgenden physikalischen und chemischen Eigenschaften besitzen, oder deren Salze :

TAN-749A

    (1) Erscheinungsbild: farbloser Feststoff

    (2) Molekularformel: $C_{15}H_{27}N_5O_3 \cdot 2HCl$

    (3) UV- (Ultraviolett) Absorptionsspektrum (in Wasser):

$$\lambda_{max} \ 266 \pm 3 \ nm \ (E^{1\%}_{1cm} = 658 \pm 100)$$

    (4) Infrarot- (IR) Absorptionsspektrum (KBr-Methode):

Die Hauptwellenzahlen sind wie folgt:

3400, 3100, 1700, 1670, 1550, 1440, 1380, 1340, 1280, 1220, 1150, 1100, 1000, 960, 870, 680 $(cm^{-1})$

    (5) $^{13}$C-Kernmag netisches Resonanz- (NMR) Spektrum:

Die folgenden Signale sind in Deuteriumoxid bei 100 MHz gemessen:

174,7 (s), 172,3 (s), 171,7 (s), 139,3 (d), 139,3 (d), 129,6 (d), 125,3 (d), 69,2 (d), 49,2 (d), 47,8 (t), 39,8 (t), 39,1 (t), 38,1 (t), 35,2 (t), 16,0 (q) (ppm)

    (6) Farbreaktion:

        Positiv:      Ehrlich-Reaktion, Dimethylbenzaldehyd, Kaliumpermanganat

        Negativ:     Ninhydrin, Greig-Leaback-, Sakaguchi-, Dragendorff-Reaktion

TAN-749B

    (1) Erscheinungsbild: farbloser Feststoff

    (2) Molekularformel: $C_{16}H_{29}N_5O_3 \cdot 2HCl$

    (3) UV-Spektrum (in Wasser):

$$\lambda_{max} \ 264 \pm 3 \ nm \ (E^{1\%}_{1cm} = 660 \pm 100)$$

(4) IR-Spektrum (KBr-Methode):

3300, 3100, 1700, 1660, 1610, 1550, 1450, 1420, 1380, 1350, 1280, 1220, 1150, 1070, 1000, 960, 930, 870, 690 (cm$^{-1}$)

(5) $^{13}$C-NMR-Spektrum:

Die folgenden Signale sind in Deuteriumoxid bei 100 MHz gemessen:

174,7 (s), 171,6 (s), 139,3 (d), 139,2 (d), 129,6 (d), 125,5 (d), 72,6 (d), 52,3 (d), 49,3 (d), 39,9 (t), 39,1 (t), 37,5 (t), 35,2 (t), 18,8 (q), 16,0 (q) (ppm)

(6) Farbreaktion:

Positiv: Ehrlich-Reaktion, Dimethylbenzaldehyd, Kaliumpermanganat

Negativ: Ninhydrin, Greig-Leaback-, Sakaguchi-, Dragendorff-Reaktion

TAN-749C

(1) Erscheinungsbild: farbloser Feststoff

(2) Molekularformel: $C_{15}H_{27}N_5O_3*2HCl$

(3) UV-Spektrum (in Wasser):

$$\lambda\text{ max } 262 \pm 3 \text{ nm } (E^{1\%}_{1cm} = 680 \pm 100)$$

(4) IR-Spektrum (KBr-Methode):

3250, 3070, 1660, 1630, 1540, 1430, 1340, 1260, 1200, 1150, 1085, 995, 860, 650 (cm$^{-1}$)

(5) $^{13}$C-NMR-Spektrum:

Die folgenden Signale sind in Deuteriumoxid bei 100 MHz gemessen:

174,7 (s), 172,3 (s), 171,6 (s), 145,1 (d), 143,0 (d), 132,0 (d), 123,1 (d), 69,2 (d), 49,2 (d), 47,7 (t), 39,7 (t), 39,1 (t), 38,0 (t), 35,2 (t), 20,6 (q) (ppm)

(6) Farbreaktion:

Positiv: Ehrlich-Reaktion, Dimethylbenzaldehyd, Kaliumpermanganat

Negativ: Ninhydrin, Greig-Leaback-, Sakaguchi-, Dragendorff-Reaktion

TAN-749D

(1) Erscheinungsbild: farbloser Feststoff

(2) Molekularformel: $C_{16}H_{29}N_5O_3*2HCl$

(3) UV-Spektrum (in Wasser):

$$\lambda\text{ max } 262 \pm 3 \text{ nm } (E^{1\%}_{1cm} = 655 \pm 100)$$

(4) IR-Spektrum (KBr-Methode):

3250, 3050, 1660, 1635, 1530, 1435, 1345, 1260, 1200, 1150, 995, 860, 650 (cm$^{-1}$)

(5) $^{13}$C-NMR-Spektrum:

Die folgenden Signale sind in Deuteriumoxid bei 100 MHz gemessen:

174,7 (s), 171,7 (s), 171,6 (s), 145,3 (d), 143,0 (d), 132,0 (d), 123,2 (d), 72,5 (d), 52,2 (d), 49,3 (d), 39,9 (t), 39,2 (t), 37,5 (t), 35,3 (t), 20,7 (q), 18,8 (q) (ppm)

(6) Farbreaktion:

Positiv: Ehrlich-Reaktion, Dimethylbenzaldehyd, Kaliumpermanganat

Negativ: Ninhydrin, Greig-Leaback-, Sakaguchi-, Dragendorff-Reaktion,

welches das Inkubieren eines zur Gattung Pseudomonas gehörenden, zur Produktion eines oder mehrerer der Antibiotika TAN-749A, B, C oder D befähigten Mikroorganismus in einem Medium unter Erhalt und Anreicherung eines oder mehrerer der Antibiotika TAN-749A, B, C oder D in der Kulturbrühe und darauf seine (ihre) Gewinnung umfaßt

**2.** Verfahren wie in Anspruch 1 beansprucht, worin der Mikroorganismus Pseudomonas fluorescens ist.

**3.** Verfahren wie in Anspruch 1 beansprucht, worin der Mikroorganismus Pseudomonas fluorescens YK-437 (FERM BP-1005) ist.

**Revendications**

**Revendications pour les Etats contractants suivants : BE, CH, LI, DE, FR, GB, IT, LU, NL, SE**

1. Antibiotiques TAN-749A, B, C et D ou leurs sels, lesdits antibiotiques présentant, sous forme de dichlorhydrates, les propriétés physiques et chimiques suivantes :

TAN-749A

(1) Aspect : solide incolore

(2) Formule brute : $C_{25}H_{27}N_5O_3.2HCl$

(3) Spectre d'absorption dans l'ultraviolet (UV) (dans l'eau) :

$$\lambda_{max} : 266 \pm 3 \text{ nm } (E_{1cm}^{1\%} = 658 \pm 100)$$

(4) Spectre d'absorption dans l'infrarouge (IR) (méthode au KBr) :

Les nombres d'onde principaux sont les suivants :

3400, 3100, 1700, 1670, 1550, 1440, 1380, 1340, 1280, 1220, 1150, 1100, 1000, 960, 870, 680 $(cm^{-1})$

(5) Spectre de résonance magnétique nucléaire (RMN) de $^{13}C$ :

On observe les signaux suivants, dans l'oxyde de deutérium, à 100 MHz :

174,7(s), 172,3(s), 171,7(s), 139,3(d), 139,3(d), 129,6(d), 125,3(d), 69,2(d), 49,2(d), 47,8(t), 39,8(t), 39,1(t), 38,1(t), 35,2(t), 16,0(q) (ppm)

(6) Réaction avec coloration :

Réponse positive : réaction d'Ehrlich, diméthylbenzaldéhyde, permanganate de potassium

Réponse négative : réaction à la ninhydrine, réaction de Greig-Leaback, réaction de Sakaguchi, réaction de Dragendorff

TAN-749B

(1) Aspect : solide incolore

(2) Formule brute : $C_{16}H_{29}N_5O_3.2HCl$

(3) Spectre UV (dans l'eau) :

$$\lambda_{max} : 264 \pm 3 \text{ nm } (E_{1cm}^{1\%} = 660 \pm 100).$$

(4) Spectre IR (méthode au KBr) :

3300, 3100, 1700, 1660, 1610, 1550, 1450, 1420, 1380, 1350, 1280, 1220, 1150, 1070, 1000, 960, 930, 870, 690 $(cm^{-1})$

(5) Spectre RMN de $^{13}C$:

On observe les signaux suivants, dans l'oxyde de deutérium, à 100 MHz :

174,7(s), 171,6(s), 139,3(d), 139,2(d), 129,6(d), 125,5(d), 72,6(d), 52,3 (d), 49,3(d), 39,9(t), 39,1(t), 37,5(t), 35,2(t), 18,8(q), 16,0(q) (ppm)

(6) Réaction avec coloration :

Réponse positive : réaction d'Ehrlich, diméthylbenzaldéhyde, permanganate de potassium

Réponse négative : réaction à la ninhydrine, réaction de Greig-Leaback, réaction de Sakaguchi, réaction de Dragendorff

TAN-749C

(1) Aspect : solide incolore

(2) Formule brute : $C_{15}H_{27}N_5O_3.2HCl$

(3) Spectre UV (dans l'eau) :

$$\lambda_{max} : 262 \pm 3 \text{ nm } (E_{1cm}^{1\%} = 680 \pm 100)$$

(4) Spectre IR (méthode au KBr) :

3250, 3070, 1660, 1630, 1540, 1430, 1340, 1260, 1200, 1150, 1085, 995, 860, 650 $(cm^{-1})$

(5) Spectre RMN de $^{13}C$ :

On observe les signaux suivants, dans l'oxyde de deutérium, à 100 MHz :

174,7(s), 172,3(s), 171,6(s), 145,1(d), 143,0(d), 132,0(d), 123,1(d), 69,2(d), 49,2(d), 47,7(t), 39,7(t), 39,1(t), 38,0(t), 35,2(t), 20,6(q) (ppm)

(6) Réaction avec coloration :

Réponse positive : réaction d'Ehrlich, diméthylbenzaldéhyde, permanganate de potassium

Réponse négative : réaction à la ninhydrine, réaction de Greig-Leaback, réaction de Sakaguchi, réaction de Dragendorff

TAN-749D

(1) Aspect : solide incolore

(2) Formule brute : $C_{16}H_{29}N_5O_3 \cdot 2HCl$

(3) Spectre UV (dans l'eau) :

$$\lambda_{max} : 262 \pm 3 \text{ nm } (E_{1cm}^{1\%} = 655 \pm 100)$$

(4) Spectre IR (méthode au KBr) :

3250, 3050, 1660, 1635, 1530, 1435, 1345, 1260, 1200, 1150, 995, 860, 650 $(cm^{-1})$

(5) Spectre RMN de $^{13}C$ :

On observe les signaux suivants, dans l'oxyde de deutérium, à 100 MHz :

174,7(s), 171,7(s), 171,6(s), 145,3(d), 143,0(d), 132,0(d), 123,2(d), 72,5(d), 52,2(d), 49,3(d), 39,9(t), 39,2(t), 37,5(t), 35,3(t), 20,7(q), 18,8(q) (ppm)

(6) Réaction avec coloration :

Réponse positive : réaction d'Ehrlich, diméthylbenzaldéhyde, permanganate de potassium

Réponse négative : réaction à la ninhydrine, réaction de Greig-Leaback, réaction de Sakaguchi, réaction de Dragendorff

**2.** Composé selon la revendication 1, qui est le dichlorhydrate de l'antibiotique TAN-749A, B, C ou D.

**3.** Procédé de production des antibiotiques TAN-749A, B, C et D tels que définis dans la revendication 1, ou de leurs sels, qui comprend l'incubation d'un microbe appartenant au genre Pseudomonas, capable de produire un ou plusieurs des antibiotiques TAN-749A, B, C et D, dans un milieu, pour produire et accumuler un ou plusieurs des antibiotiques TAN-749 A, B, C et D dans le bouillon de culture et pour récupérer ensuite le ou les antibiotiques.

**4.** Procédé selon la revendication 3, dans lequel le microbe est Pseudomonas fluorescens.

**5.** Procédé selon la revendication 3, dans lequel le microbe est Pseudomonas fluorescens YK-437 (FERM BP-1005).

**6.** Composition pharmaceutique pour le traitement des infections bactériennes, qui contient une quantité efficace d'un ou de plusieurs des antibiotiques TAN-749A, B, C et D tels que définis dans la revendication 1, et un excipient pharmacologiquement acceptable.

**7.** Culture biologiquement pure de Pseudomonas fluorescens YK-437 (FERM BP-1005) et de ses mutants et variants, capable de produire un ou plusieurs des antibiotiques TAN-749A, B, C et D tels que définis dans la revendication 1.

## Revendications pour l'Etat contractant suivant : AT

**1.** Procédé de production des antibiotiques TAN-749A, B, C et D ou de leurs sels, lesdits antibiotiques présentant, sous forme de dichlorhydrates, les propriétés physiques et chimiques suivantes :

TAN-749A

(1) Aspect : solide incolore

(2) Formule brute : $C_{15}H_{27}N_5O_3 \cdot 2HCl$

(3) Spectre d'absorption dans l'ultraviolet (UV) (dans l'eau) :

$$\lambda_{max} : 266 \pm 3 \text{ nm } (E_{1cm}^{1\%} = 658 \pm 100)$$

(4) Spectre d'absorption dans l'infrarouge (IR) (méthode au KBr) :

Les nombres d'onde principaux sont les suivants :

3400, 3100, 1700, 1670, 1550, 1440, 1380, 1340, 1280, 1220, 1150, 1100, 1000, 960, 870, 680 $(cm^{-1})$

(5) Spectre de résonance magnétique nucléaire (RMN) de $^{13}C$ :

On observe les signaux suivants, dans l'oxyde de deutérium, à 100 MHz :
174,7(s), 172,3(s), 171,7(s), 139,3(d), 139,3(d), 129,6(d), 125,3(d), 69,2(d), 49,2(d), 47,8(t), 39,8(t), 39,1(t), 38,1(t), 35,2(t), 16,0(q) (ppm)

(6) Réaction avec coloration :

Réponse positive : réaction d'Ehrlich, diméthylbenzaldéhyde, permanganate de potassium

Réponse négative : réaction à la ninhydrine, réaction de Greig-Leaback, réaction de Sakaguchi, réaction de Dragendorff

TAN-749B

(1) Aspect : solide incolore

(2) Formule brute : $C_{16}H_{29}N_5O_3.2HCl$

(3) Spectre UV (dans l'eau) :

$$\lambda_{max} : 264 \pm 3 \ nm \ (E_{1cm}^{1\%} = 660 \pm 100)$$

(4) Spectre IR (méthode au KBr) :
3300, 3100, 1700, 1660, 1610, 1550, 1450, 1420, 1380, 1350, 1280, 1220, 1150, 1070, 1000, 960, 930, 870, 690 $(cm^{-1})$

(5) Spectre RMN de $^{13}C$ :

On observe les signaux suivants, dans l'oxyde de deutérium, à 100 MHz :
174,7(s), 171,6(s), 139,3(8), 139,2(d), 129,6(d), 125,5(d), 72,6(d), 52,3 (d), 49,3(d), 39,9(t), 39,1(t), 37,5(t), 35,2(t), 18,8(q), 16,0(q) (ppm)

(6) Réactton avec coloration :

Réponse positive : réaction d'Ehrlich, diméthylbenzaldéhyde, permanganate de potassium

Réponse négative : réaction à la ninhydrine, réaction de Greig-Leaback, réaction de Sakaguchi, réaction de Dragendorff

TAN-749C

(1) Aspect : solide incolore

(2) Formule brute : $C_{15}H_{27}N_5O_3.2HCl$

(3) Spectre UV (dans l'eau) :

$$\lambda_{max} : 262 \pm 3 \ nm \ (E_{1cm}^{1\%} = 680 \pm 100)$$

(4) Spectre IR (méthode au KBr) :
3250, 3070, 1660, 1630, 1540, 1430, 1340, 1260, 1200, 1150, 1085, 995, 860, 650 $(cm^{-1})$

(5) Spectre RMN de $^{13}C$ :

On observe les signaux suivants, dans l'oxyde de deutérium, à 100 MHz :
174,7(s), 172,3(s), 171,6(s), 145,1(d), 143,0(d), 132,0(d), 123,1(d), 69,2(d), 49,2(d), 47,7(t), 39,7(t), 39,1(t), 38,0(t), 35,2(t), 20,6(q) (ppm)

(6) Réaction avec coloration :

Réponse positive : réaction d'Ehrlich, diméthylbenzaldéhyde, permanganate de potassium

Réponse négative : réaction à la ninhydrine, réaction de Greig-Leaback, réaction de Sakaguchi, réaction de Dragendorff

TAN-749D

(1) Aspect : solide incolore

(2) Formule brute : $C_{16}H_{29}N_5O_3.2HCl$

(3) Spectre UV (dans l'eau) :

$$\lambda_{max} : 262 \pm 3 \ nm \ (E_{1cm}^{1\%} = 655 \pm 100)$$

(4) Spectre IR (méthode au KBr) :
3250, 3050, 1660, 1635, 1530, 1435, 1345, 1260, 1200, 1150, 995, 860, 650 $(cm^{-1})$

(5) Spectre RMN de $^{13}C$ :

On observe les signaux suivants, dans l'oxyde de deutérium, à 100 MHz :
174,7(s), 171,7(s), 171,6(s), 145,3(d), 143,0(d), 132,0(d), 123,2(d), 72,5(d), 52,2(d), 49,3(d), 39,9(t), 39,2(t), 37,5(t), 35,3(t), 20,7(q), 18,8(q) (ppm)

24

(6) Réaction avec coloration :

Réponse positive : réaction d'Ehrlich, diméthylbenzaldéhyde, permanganate de potassium

Réponse négative : réaction à la ninhydrine, réaction de Greig-Leaback, réaction de Sakaguchi, réaction de Dragendorff,

procédé qui comprend l'incubation d'un microbe appartenant au genre Pseudomonas, capable de produire un ou. plusieurs des antibiotiques TAN-749A, B, C et D, dans un milieu, pour produire et accumuler un ou plusieurs des antibiotiques TAN-749A, B, C et D dans le bouillon de culture et pour récupérer ensuite le ou les antibiotiques.

2.   Procédé selon la revendication 1, dans lequel le microbe est Pseudomonas fluorescens.

3.   Procédé selon la revendication 1, dans lequel le microbe est Pseudomonas fluorescens YK-437 (FERM BP-1005).

Fig. 1

Transmittance (%)

Wave number $(cm^{-1})$

Fig. 2

PPm

Fig. 3

Fig. 4

Fig. 5

Fig. 6

Fig. 7

Fig. 8

Fig. 9

Fig. 10